# EUROPEAN PATENT APPLICATION

(11) **EP 4 678 301 A1**
(43) Date of publication of application: **14.01.2026**
(21) Application number: 24188091.3
(22) Date of filing: 11.07.2024
(51) Int. Cl.: B07C 5/342, D01G 99/00, G01N 33/36, G06T 7/00, G01N 21/84

(54) **METHOD OF COTTON FIBER CLASSIFICATION**

(71) Applicant: Rieter AG, 8406 Winterthur (CH)
(72) Inventor: Wolfer, Tobias, 8595 Altnau (CH); Paganucci, Silvio, 6003 Luzern (CH); Vera, Benjamin, 6048 Horw (CH); Eberle, Patric, 4423 Hersberg (CH)
(74) Representative: Rieter

(57) **Abstract**

The present invention concerns a method (500) of retracing of fiber in a fiber layer (900). Said fiber layer (900) is spread on a surface such as to acquire (510) an image (511) of said fiber layer (900) comprising a plurality of fibers (910) including at least one fiber (911). At least one image-filter (531) is applied to said acquired image (511) and a at least one list (120) is created (570) per said at least one fiber (911) such as to classify said fiber.

## Description

### Technical field of the invention

The present invention relates to a method of classification of fiber. More particularly, the present invention may be dedicated, but not exclusively, to the classification of a cotton fiber in a fiber layer, wherein said fiber layer may comprise at least one fiber and preferably a plurality of fiber. Preferably, the present invention relates to measuring the length and characterizing the cotton fibers.

### Technological background

Several factors may affect how cotton or other fibers are graded or classed. For example, some of the factors that affect the grade assigned to cotton fibers are the length of the cotton fibers, the tensile strength of the cotton fibers, the color of the fibers, the characteristics of the fiber and the dust content of the cotton. Indeed, the proportion of short fibers may have a very substantial influence on some parameters.

Besides this influence, a large proportion of short fibers may also lead to considerable fly contamination among other problems for example, and thus to strain on personnel, on the machines, on the workroom, and on the air-conditioning.

Further, the proportion of short fibers has increased substantially in recent years in cotton available from many sources. This may be due to mechanical picking and hard ginning. In some of cases, the absolute short-fiber proportion may specified today as the percentage of fibers shorter than 10, 11, 13 or 13.5 mm, for instance. The short-fiber limit may not have been standardized but may settle at around 13 or 13.5 mm. Since the short fibers cannot be measured easily, this value is seldom really accurate.

Apparatuses for measuring characteristics of cotton fibers are known, which are provided with a preparation device for preparing the sample of fibers to be tested, wherein the sample picked up by a user is in the form of a "beard". The procedure is, however, very demanding and one of the disadvantages here is that only an estimated quantitative analysis of the fraction of the waste present in the available fiber mass is obtained. A more accurate quantitative analysis or even a qualitative analysis of the components contained in the fiber mass is not possible.

An objective of the present invention is to retrace a fiber such as to calculate the total length of each individual fibers whilst allowing a quantitative, above all, qualitative analysis by collecting the characteristics of said retraced fiber for potential statistic.

### Summary of the invention

In order to achieve this objective, the present invention may provide, according to an aspect of the invention, a method of classification of cotton fiber in a fiber layer; said fiber layer is spread on a surface such as to acquire an image of said fiber layer comprising a plurality of fibers including at least one fiber; said method comprising a/an:
- Application of at least one image-filter to said acquired image;
- Representation of said at least one fiber comprising a set of characteristics by a group of segments comprising at least one segment, at least one section and/or at least one structural feature;
- Creation of at least one list per said set of characteristics, per said at least one segment, per said at least one section, and/or per said at least one structural feature; and,
- Incrementation of said at least one list for each occurrence of said set of characteristics, said at least one segment, said at least one section, and/or said at least one structural feature.

Thanks to this configuration, said method may classify said fiber from said acquired image.

According to an embodiment, said method comprises a length calculation; said length calculation calculates the length of said at least one segment and/or of said at least one section of said at least one list.

Advantageously, said method may calculate the length of said fiber.

According to an embodiment, said method comprises a concatenation of said at least one list in accordance with said set of characteristics such as to calculate the length of said at least one fiber.

Advantageously, said method may calculate the total length of said fiber.

According to an embodiment, said method comprises a selection of the two longest lengths of said at least one lists.

Advantageously, said method may discriminate the branching of said fiber in order to calculate the total length of said fiber.

According to an embodiment, said method comprises a mean calculation of said at least one fiber, of said group of segments, of said at least one segment, of said at least one section, of said at least one structural feature, of said endpoint, of said vertex and/or of said junction, of said at least one segment, of said length of said at least one fiber, of said length of said group of segments, of said length of said at least one segment, of said length of said at least one section and/or of said length of at least one segment.

Advantageously, said method may depict a representation of said set of characteristics and/or or said at least one structural feature.

According to an embodiment, wherein said at least one segment comprises extremities; said extremity comprises an endpoint, a vertex or a junction; said at least one structural feature comprises:
- said endpoint: said endpoint terminates said at least one segment;
- said vertex; said extremities of two segments meet at said vertex; and/or,
- said junction: said extremities of at least three segments meet each other at said junction.

Advantageously, said method may identify sections of said fiber, via said group of segments, comprising two endpoints, an endpoint and a junction, vertices or two junctions.

According to an embodiment, said set of characteristics comprises a maturity index, a fineness, said length, a weight, a color, a waviness, a width, a spinning consistency, a uniformity index, a short fiber index, an elongation, a moisture, a reflectance, a yellowness and/or a strength.

Advantageously, said method may characterize said at least one fiber.

### Brief description of the drawings

The foregoing and other purposes, features, aspects and advantages of the invention will become apparent from the following detailed description of embodiments, given by way of illustration and not limitation with reference to the accompanying drawings, in which the same reference refer to similar elements or to elements having similar functions, and in which:
- Figure 1 may represent an acquired image **511** of a fiber layer **900** comprising a plurality of fibers **910** spread on a surface;
- Figure 2 may illustrate the application **530** of at least one image-filter **531** to said acquired image **511;**
- Figure 3 may show the representation **540** said at least one fiber **911** by a group of segments **100;**
- Figures 4-7 may depict the different connections **150, 151** and endpoint of an extremity;
- Figure 8 may represent the classification of said extremities in different categories;
- Figure 9 may represent the incrementation **520** of a set of characteristics **125** and/or a or said at least one structural feature;
- Figure 10 may depict the referencing **560** of at least one list **120, 121, 122;**
- Figure 11 may illustrate a length calculation **595** and/or a concatenation **596** of said at least one list **120, 121, 122;**
- Figures 12A & 12B may represent an example of a black and white acquired image **511** and said representation **540** of said fibers **911** by said group of segments **100, 101;**
- Figures 13-15 may show different retraced said at least one fiber **911** with pseudocolors; and,
- Figures 16A-16B may illustrate a representation of length by weight and length by number.

In the figures and in the remainder of the description, the same references represent identical or similar elements. In addition, the various elements are not represented to scale so as to favor the clarity of the figures. Furthermore, the different embodiments and variants are not mutually exclusive and can be combined with one another.

### Detailed description

Raw material represents about 50-75% of the manufacturing cost of a short-staple yarn. This fact alone is sufficient to indicate the significance of the raw material for the yarn producer. The influence becomes still more apparent when the ease in processing one type of fiber material is compared with the difficulties, annoyance, additional effort, and the decline in productivity and quality associated with another similar material. But hardly any spinner can afford to use a problem-free raw material because it would normally be too expensive. Adapting to the expected difficulties requires an intimate knowledge of the starting material and its behavior in processing and subsequent stages. Optimal conditions can be obtained only through mastery of the raw material: this is one of the objectives that the present invention may address.

There is a further difficulty: Cotton is a natural fiber **911.** It grows in various soils, in various climates, and with annually changing cultivation conditions. The fibers **911** therefore cannot be homogeneous in their characteristics. The cotton fiber **911** may comprise cell wall and lumen. The maturity index, which may belong to a set of characteristics **125** of the fiber **911,** may dependent upon the thickness of this cell wall. A fiber **911** may be considered as mature when the cell wall of the moisture-swollen fiber represents 50-80% of the round cross-section, as immature when it represents 30-45%, and as dead when it represents less than 25%.

Since some 5% immature fibers are present even in a fully matured boll, cotton stock without immature fibers is unimaginable: the quantity is the issue. The International Textile Manufacturers Federation, ITMF for short, recommends the Fiber Maturity Tester for cotton maturity determination. The Immature fibers have neither adequate strength nor adequate longitudinal stiffness, which both may belong to said set of characteristics **125,** they therefore lead to loss of yarn strength, nippiness, a high proportion of short fibers, varying dyeability and/or processing difficulties mainly at the card for example.

Fineness may be one of the most important fiber characteristics in said set of characteristics **125.** A multitude of fibers **911** in the cross-section provide not only high strength, which may belong to said set of characteristics **125,** but also better distribution in the yarn. The fineness determines how many fibers are present in the cross-section of a yarn of given thickness. Additional fibers in the cross-section provide not only additional strength but also better evenness in the yarn. About thirty fibers are needed at the minimum in the yarn cross-section, but there are usually over one hundred. This number is approximately the lower limit for almost all new spinning processes, in other words, this indicates that fineness may be one the most important characteristic of said set of characteristics **125** since fineness may influence the spinning limit, the yarn strength, the yarn evenness, the yarn fullness, the drape of the fabric, the luster, the handle and/or the productivity of the process.

Fiber fineness of cotton may not be specified by reference to diameter as in the case of steel wire for example, because the cross-section may be seldom circular and may be thus not easily measurable, but the width of the fiber may be measured and may belong to said set of characteristics **125.** As in the case of yarns and fibers, fineness may be usually specified by the relation of mass, i.e. weight, which may belong to said set of characteristics, to length **124** like the measurement unit "tex", which may belong to said set of characteristics and may be the ration between mass in gram and the length **124** in kilometer.

Therefore, said fiber length **124** may also be one of the most important fiber characteristics of said set of characteristics **125.** It may influence the spinning limit, the yarn strength, the yarn evenness, the handle of the product, the luster of the product, the yarn hairiness and/or the productivity for example. It may be assumed that fibers of under 4-5 mm will be lost in processing as waste and/or fly, fibers up to about 13-15 mm may not contribute much to strength but only to fullness of the yarn, and only those fibers above these lengths **124** may produce the other positive characteristics in the yarn.

Indeed, the proportion of short fibers may have a very substantial influence on some parameters listed previously. Besides this influence, a large proportion of short fibers may also lead to considerable fly contamination among other problems for example, and thus to strain on personnel, on the machines, on the workroom, and on the air-conditioning, and also to extreme drafting difficulties for example, and this may affect the price of the raw material for example. Unfortunately, the proportion of short fibers has increased substantially in recent years in cotton available from many sources. This may be due to mechanical picking and hard ginning. In some of cases, the absolute short-fiber proportion may be specified today as the percentage of fibers shorter than 10, 11, 13 or 13.5 mm, for instance. The short-fiber limit may not have been standardized but may settle at around 13 or 13.5 mm. According to an embodiment, the present invention may determine a more accurate percentage of short fibers and said set of characteristics **125,** which may comprise said maturity index, said fineness, said length, a weight, the color of the fiber, a waviness, said width, a spinning consistency, an uniformity index, a short fiber index, an elongation, a moisture, a reflectance, a yellowness and/or the strength for example.

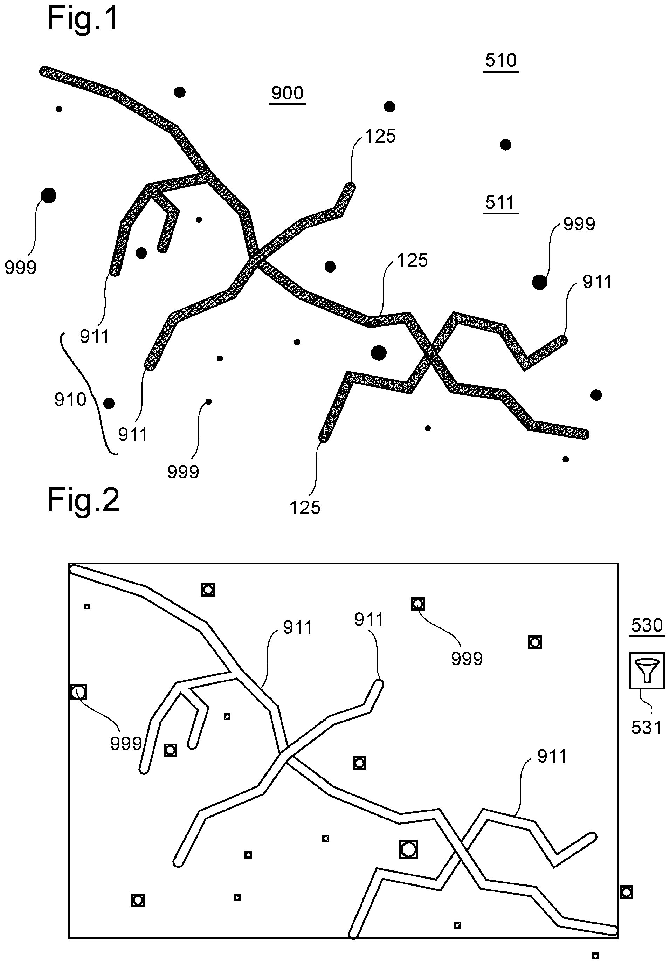

In order to measure said length **124** of the fiber, a plurality of fibers **910** may be spread on a surface and may form a fiber layer **900.** In said fiber layer **900,** said fibers **911** may be made from staple fibers **911** and/or long fibers **911,** wherein said staple fibers **911** and/or long fibers **911** may cross some fibers **911** and/or may be distant from some fibers **911** as illustrated in Fig. 1.

In example of fig. 1, an image **511** may comprise a plurality of fibers **910,** namely three fibers **911,** and a plurality of dusts **999,** which may be seeds, grain of sand, trash, plant residues and other contaminants for example. Said image **511** may be acquired **510,** i.e. a picture **511** may have been taken of said fibers layer **900** via a camera for example. Alternatively, said image **511** may comprise a plurality of pictures **511** having at least one picture **511** and may be sent by uploading said image **511** from said camera or from a remote device, like computer, camera or handheld device for example, onto a file-hosting service via wire transmission and/or via wireless transmission. A method **500** of classification of fiber in said fiber layer **900,** subject matter of the present invention, may open or receive said image **511** and may model **540** or represent **540** said fiber **911** so that said fiber **911** may be characterized to obtain some features of fiber, like said length **124** of the fiber, said maturity index, said fineness, said length, said weight, said color, said waviness, said width, said spinning consistency, said uniformity index, said short fiber index, said elongation, said moisture, said reflectance, said yellowness and/or said strength for example.

Said method **500** may reconstruct or retrace said fiber, by means of said image **511.** For this purpose, said method **500** may apply **530** of at least one image-filter **531** to said acquired image **511,** as illustrated in fig. 2. Said image-filter **531** may process said acquired images **511** through an algorithm like Thresholding, Clustering, Motion and interactive segmentation, Gaussian-Blurring-Filters, Compression-based methods, Histogram-based methods, Edge detection, Dual clustering method, Image destriping, Region-growing methods, Acutance, Partial differential equation-based methods, Iterative reconstruction, Toggle Partial differential equation-based methods subsection, Topological skeleton, Inpainting, Variational methods, Graph partitioning methods, Watershed transformation, Image stitching, Model-based segmentation and/or Multi-scale segmentation to name a few of them. In some case, a best result may be achieved by using Thresholding, Clustering, Gaussian-Blurring-Filters, Histogram-based methods, Edge detection, Image destriping, Region-growing methods, Acutance, Iterative reconstruction, Topological skeleton, Inpainting, Variational methods, Graph partitioning methods, Image stitching, Model-based segmentation and/or Multi-scale segmentation for example.

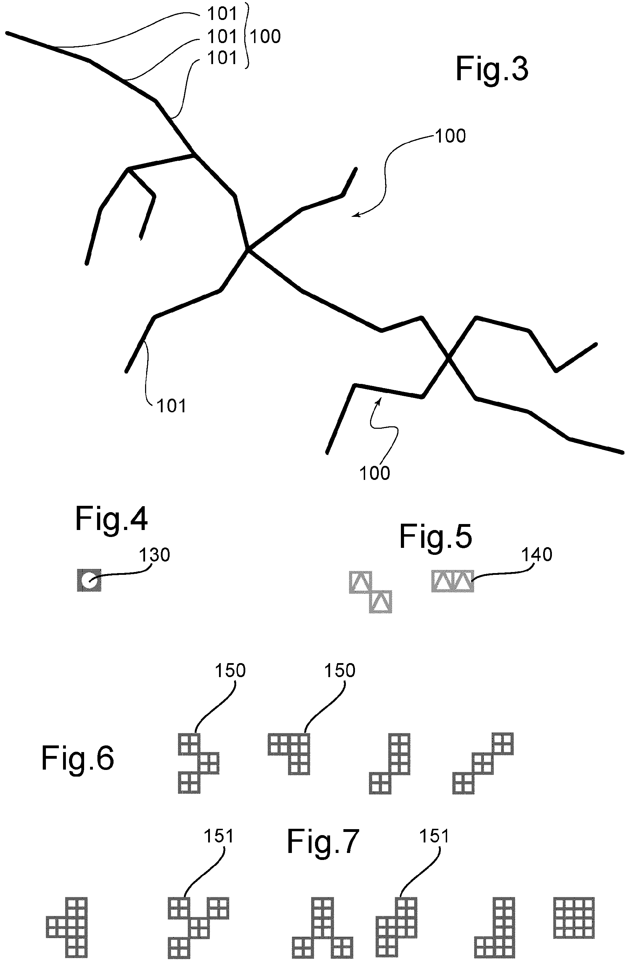

Said at least one image-filter **531** may filter out the dust **999** as illustrated in fig. 3 and may isolate some entangled fibers **911** as an island, such as to facilitate said image processing for example. Although said dust **999** may be filtered out at this stage, it may remain important for the spinning mill to know that said dust **999** may comprise small and microscopic particles of various substances, which may be present in fiber layer **900** and may be released during the processing as suspended particles in the air, which may be transported over substantial distances and may induce allergies, respiratory disease like byssinosis, contamination of the air-conditioning, quality deterioration directly and/or stress on the machines for example. For example, said dust **999** may comprise microdust **999,** ca. 50-80% fiber fragments, leaf and husk fragments, around 10-25% sand and earth, and about 10-25% water-soluble materials. The high proportion of fiber fragments may indicate that a large part of the microdust **999** may arise in the course of processing. About 40% of the microdust **999** may be free between the fibers and flocks, 20-30% may be loosely bound, and the remaining 20-30% may be firmly bound to the fibers.

Further, in addition to usable fibers, e.g. lint, cotton stock may contain foreign matter, which may be considered as dust **999** in the following description, of various kinds like vegetable matter, Mineral material, sticky contaminations, as honeydew, insect sugar, grease, oil, tar and/or additives to name few of them.

This foreign material or dust **999,** like metal fragments, cloth fragments, packing material, and/or polymer materials as example, may lead to extreme disturbances during processing. Metal parts may cause fires or damage card clothing. Cloth fragments and packing material may lead to foreign fibers in the yarn and thus to its unsuitability for the intended application, since foreign fibers may be of importance for the spinner.

Vegetable matter, like husk portions, seed fragments, stem fragments, leaf fragments and/or wood fragments for example, may lead to drafting disturbances, yarn breaks, filling-up of card clothing, contaminated yarn, etc. Mineral matter, like earth, sand, ore dust **999** picked up in transport and/or dust **999** picked up in transport for instance, may cause deposits, high wear rates in machines like grinding effects, especially apparent in rotor spinning, etc.

The new spinning processes may be very sensitive to dust **999,** i.e. foreign matter. Foreign matter, or dust **999,** was perhaps always a problem but may be becoming steadily more serious from year to year. This may be due primarily to modern high-performance picking methods, hard ginning and cleaning, pre-drying; careless handling during harvesting, packing and/or transport. For all these reasons, it may be also possible to know, thanks to this present invention, the portion of said dust **999,** or said foreign matter, present in the fiber layer **900.**

After realized said island as shown in fig. 2, said at least one image-filter **531** may aim to represent said at least one fiber **911** by a group of segments **100** having a similar color, being 1-pixel wide line, and wherein each segment may have extremities **110** for instance, as observed in fig. 3. In other words, said at least one image-filter **531** may aim to represent said at least one fiber **911** by a group of segments **100** thanks to said set of characteristics **125** as observed in fig. 3.

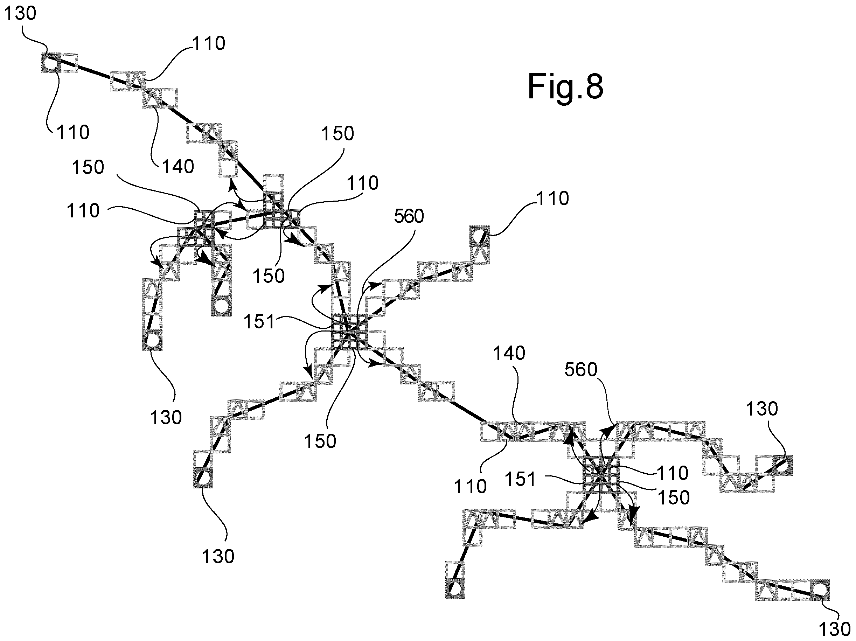

By "similar color", it may be understood that the surrounding pixels may be slightly tinted or shaded, rather the color value of the surrounding pixels of said group of segments **100** may be slightly lighter or darker. Said method **500** may classify or categorize said extremities **110** in two classes or categories: extremity with an open end and extremity with a connection. Said extremity with said open end may be terminated by an endpoint, as depicted in figs. 4 & 8, and, when two or more segments **101,** or extremities **110,** connect, that may be a vertex **140** as in figs. 5 & 8, a junction **150** like in figs. 6 & 8 or an intersection **151** as in figs. 7 & 8. For instance, said vertex **140** may be where at least two segments **101** may connect, rather at least two extremities **110** may meet and may form an angle.

Some connections may be similar to a three-pointed star, as it may be the case for said junction **150** in fig. 6, where at least three segments **101** may connect, rather at least three extremities **110** may meet and may form said three-pointed star. In some cases, said junction may comprise said intersection **151,** where at least four segments **101** may connect, rather at least four extremities **110** may meet and may form a four-pointed star, like in figs. 6 & 7, for example. In other words, said method **500** recognize said endpoint, when a pixel may have only one adjacent pixel, said junction, when a pixel may have at least three adjacent pixels, and said intersection **151,** when a pixel may have four adjacent pixels.

Thanks to this configuration, said method **500** may identify sections **102** of said fiber, via said group of segments **100,** comprising two endpoints, an endpoint, and a junction or two junctions for example. Indeed, even when a fiber may comprise, for example, two segments **101,** wherein said each segment may comprise said endpoint **130** and may share the same vertex **140,** said method **500** may identify these two segments **101** as a section.

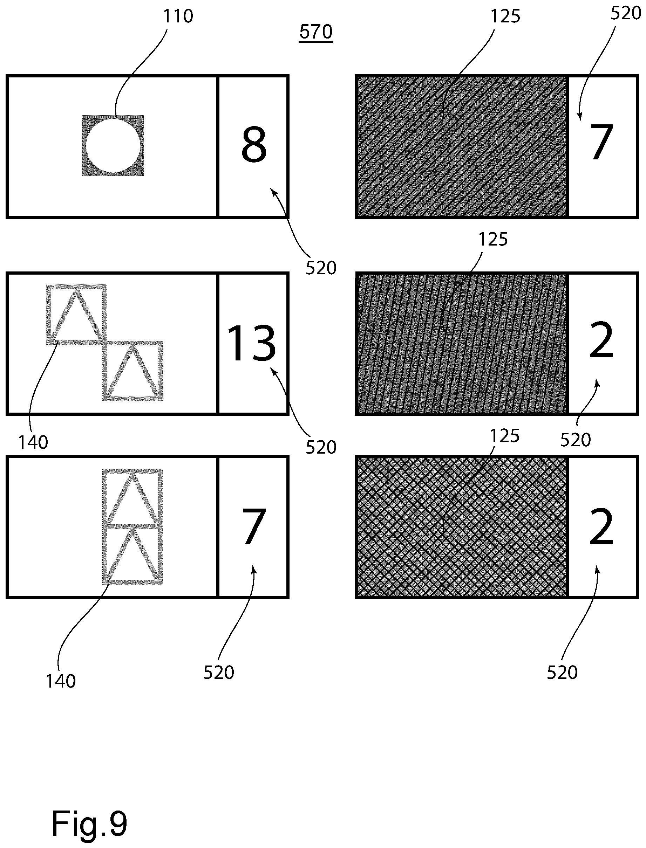

For each said at least one segment **101** or each section, said method **500** may create **570** at least one list **120** per fiber comprising a first list **121** and/or a second list **122.** Said first list **121** may comprise said at least one segment **101** or said section **102** having said endpoint and/or said junction **150,** and/or a set of characteristics **125** corresponding to said fiber. Every time when said endpoint, said junction **150,** said at least one section and/or said set of characteristics **125** may be recognized, or for each occurrence of said endpoint, said junction **150,** said at least one section and/or said set of characteristics **125** said at least one list **120, 121,** rather said first list **121** may be incremented **520,** that may mean that said method **500** may enter the number of times in said at least one list **120,** rather in said first list **120,** said at least one segment **101,** said at least one section, said at least one structural feature and/or said set of characteristics **125** may have been recognized. Fig 9 may show how some of said at least one structural feature and/or some of said set of characteristics **125** may be incremented **520** in said at least one list **120, 121** or said first list **121** for example.

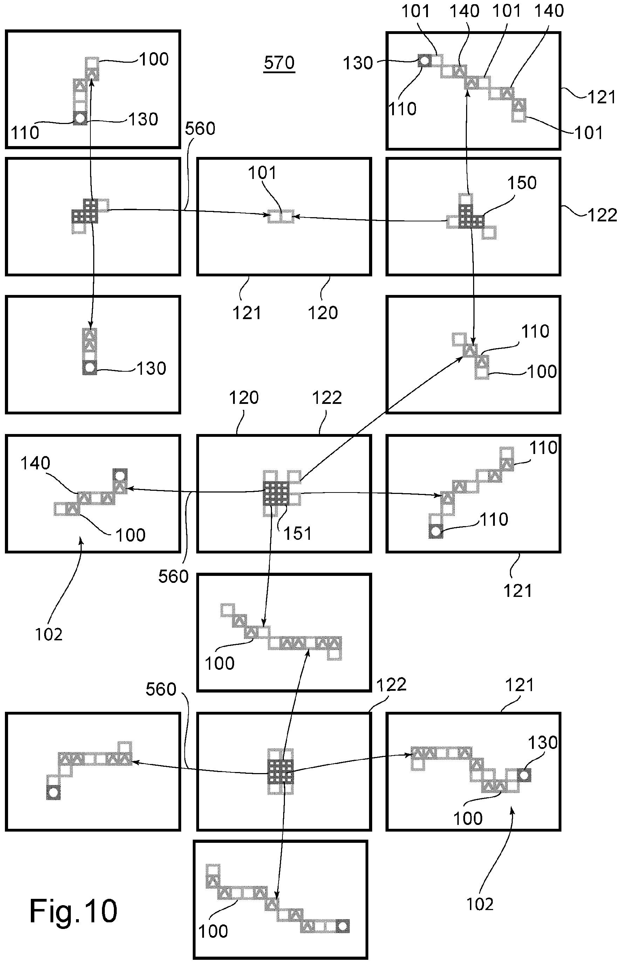

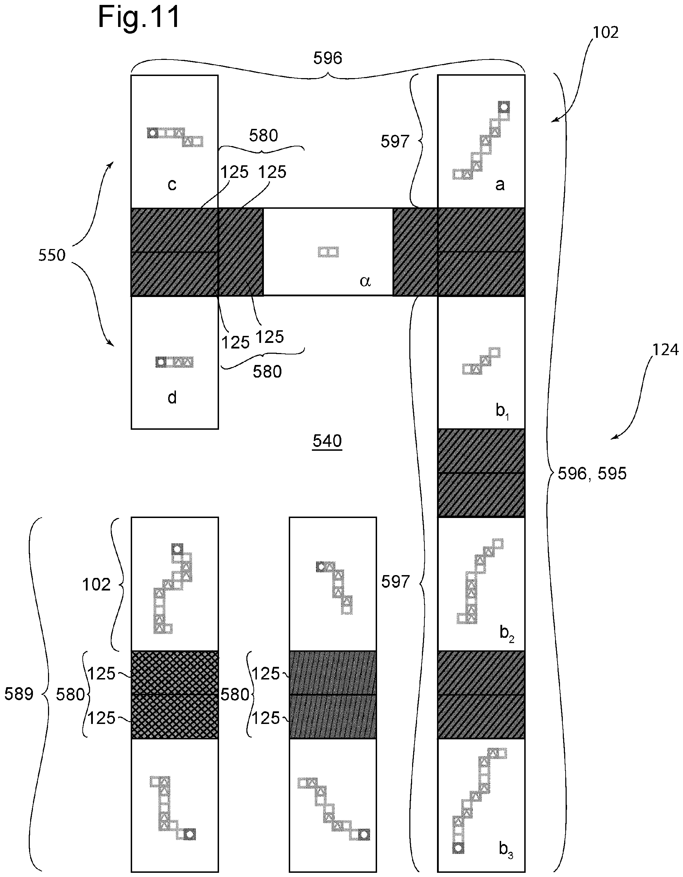

As illustrated in fig. 10, said second list **122** may refer to said first list **121** comprising said junction **150** such as to retrace **590** said fiber from said at least one list **120,** or to associate said at least one list **120** by referencing said at least one segment **101** of said first list via said second list such as to retrace **590** said fiber. More precisely, said referred first lists **121** of said second list may be linked **580** in accordance with said set of characteristics **125,** as illustrated in fig. 11, such as said method **500** may retrace **590** said fiber by associating said referred first lists **121** of said second list in accordance with said set of characteristics **125.**

After the recombination of the different section of said fiber by associating said referred first lists **121,** said retracement **590** or said association **589** may comprise a length calculation **595.** Said length calculation **595** may calculate the length **124** of said at least one segment **101** of said at least one list **120,** preferably of said first list such as to determinate the length **124** of said group of segments **100,** and in order to determinate the total length **124** of said fiber, said length calculation **595** may comprise a concatenation **596** of said first lists **121,** preferably of said first lists **121** in accordance with said set of characteristics **125.** However, since a fiber may be hairy, said method **500** may discriminate the branching of said fiber in order to calculate the total length **124** of said fiber. To that end, said length calculation **595** may comprises a selection **597** of the two longest first lists **121** referred by said second list **122,** preferably of the two longest first list comprising said at least one segment **101** having said endpoint and/or of said concatenated first lists **121.** To illustrate this selection **597,** we refer to figure 11. In this example, said concatenated fiber may have seven first lists **121,** like "a", "b₁", "b₂", "b₃", "c", "d" and "α" and two said second lists referring to said first lists **121.** To simplify this example, it may be considered, that said three first lists **121** "b₁", "b₂" and "b₃" may form only one first list "b".

Within said second list, said section or said first lists **121** may be sorted in ascending order without the segment α, since it may referred by both said second lists **122.** Finally, the two said second lists may be put together and sorted in ascending order as well: if the two longest first lists **121** are referred by the same second list, they are added together to give the total length **124** of said fiber, otherwise, if the two longest first lists **121** are referred by the different second lists, they are added together with "α" such as to give the total length **124** of said fiber. Thanks to this data, the present invention may be able to indicate the quality of said fibers, and if said fibers are Hairy-fibers, how long and in which proportion.

Indeed, said method **500** may identify **550** for each said connection, i.e. said junction or said intersection **151,** the number of segments **101** connected and if one of them is shorter than some predetermined threshold whilst having an endpoint, this segment may be identified **550** as "Hairy-Fiber" and may be considered as a characteristic of said set of characteristics **125.**

In some cases, some junctions may result from an overlap between at least two fibers, although only one section may be represented **540.** In this configuration, said method **500** may identify **550,** that among the five groups of segments **101,** two may belong to one fiber and the other two to another fiber thanks to said sets of characteristics **125** corresponding to each said fibers, and said method **500** may associate each said at list one lists such as to retrace **590** said fibers individually.

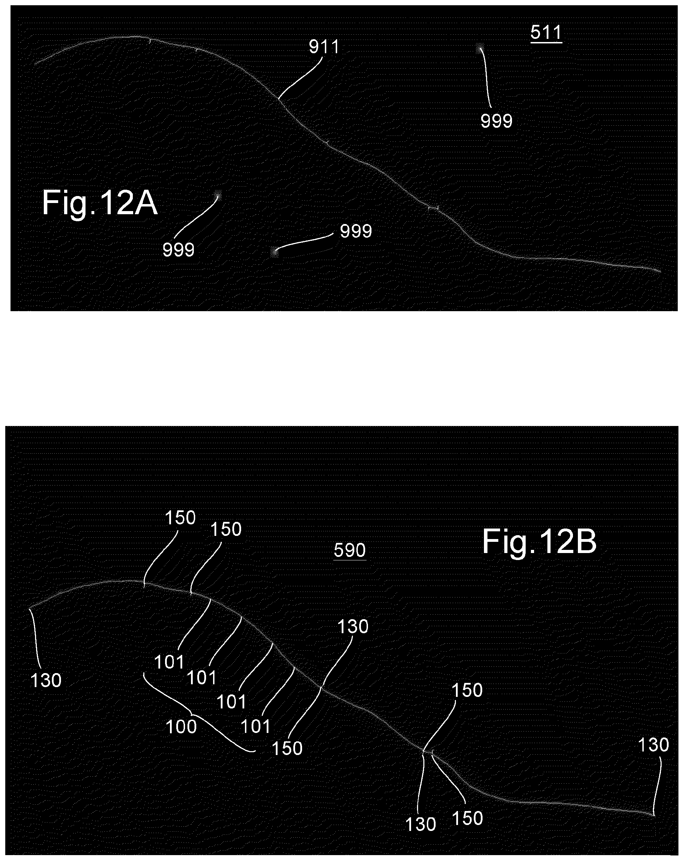

Fig. 12A shows an example of a black and white acquired image **511,** wherein said fiber layer **900** may be spread on said surface and said plurality of fibers **910** may include said at least one fiber **911.** In this acquired image **511,** it may be focused on the longest fiber for instance. After application **530** of said at least one image-filter **531** to said acquired image **511** as depict in fig. 12B, said at least one fiber **911** is represented **540** by said group of segments **100, 101,** using pseudocolors or false colors, comprising a plurality of endpoints **130** and of junctions **150.** For simplicity's sake, said first list **121** and said second list **122** of fig. 9 are used by reference.

Figs. 12A-15 may show different representation **540** of said at least one fiber **911** by said group of segments **100, 101** and by using pseudocolors. As it may be construed, said pseudocolors may be totally or partially based on said set of characteristics **125.** According to the different example, said image **511** may comprise between two and three fibers **911.**

As it may be understood, said images **511** may be a part of a plurality of pictures **511.** Departing from said acquired image **511,** said method **500** may model **540** or represent **540** said fibers **911** so that said fibers **911** may be characterized to obtain some features of fiber, like the length **124,** the proportion of hairy fiber and so on, for example. Some said at least one image-filters **531** may be applied **530** to said acquired image **511,** as illustrated in fig. 2. The result of said application **530** of said at least one image-filters **531** may be a representation in black and white said at least one fibers **911** by a group of segments **100.** For a better understanding or recognition of different said at least one fibers **911,** some pseudocolors may be added for the user.

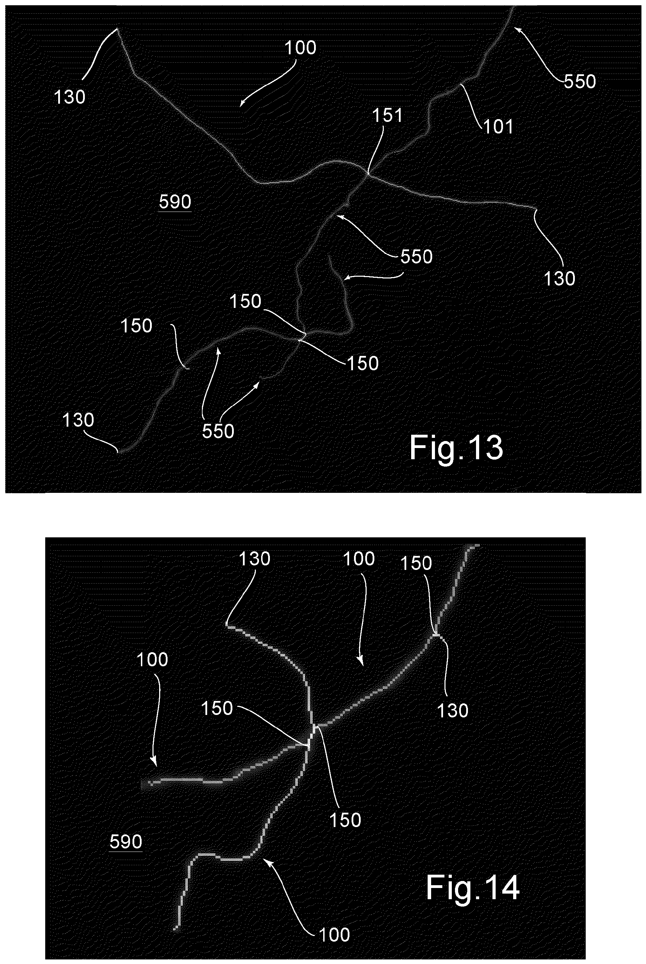

In fig. 13 for instance, it may distinguish three different said at least one fibers **911** and four connections **150, 151** including three said junctions **150** and one said intersection **151** for example. In the specific case of fig. 13, said method may differentiate two different said at least one fibers **911** with three connections **150** including three said junctions **150.** In the particular case of fig. 13, said method may recognize different connections **150, 151,** but thanks to the creation **570** of said first lists **121** having said set of characteristics **125** and said second list **122** referring to said first lists **121,** it may possible to associate said at least one lists **120** of at least one fiber **911** such as to retrace **590** individually said fiber **911** in accordance with said set of characteristics **125.** Indeed, whereas, some junctions **150** may result from an overlap between at least two fibers, as it may be particularly shown in fig. 13, only one section may be represented **540** and among the at least five groups of segments **101** identified **550,** said method **500** may recognize that two may belong to one fiber and the other two to another fiber thanks to said sets of characteristics **125.** Fig. 14 may show another example, wherein two fibers may be distinguished.

In each example of the aforementioned case, said length calculation **595** may calculate the total length **124** of each individual said fiber **911** whilst discriminating, when appropriate, the hairy fiber as previously shown, and collecting, when appropriate, said set of characteristics **125** of said retraced fiber. Thanks to this data, the present invention may be able to indicate the quality of said fibers, and if said fibers are Hairy-fiber, how long and in which proportion, and all this information may be collected as characteristic in said and may be integrated in said set of characteristics **125** for potential statistic.

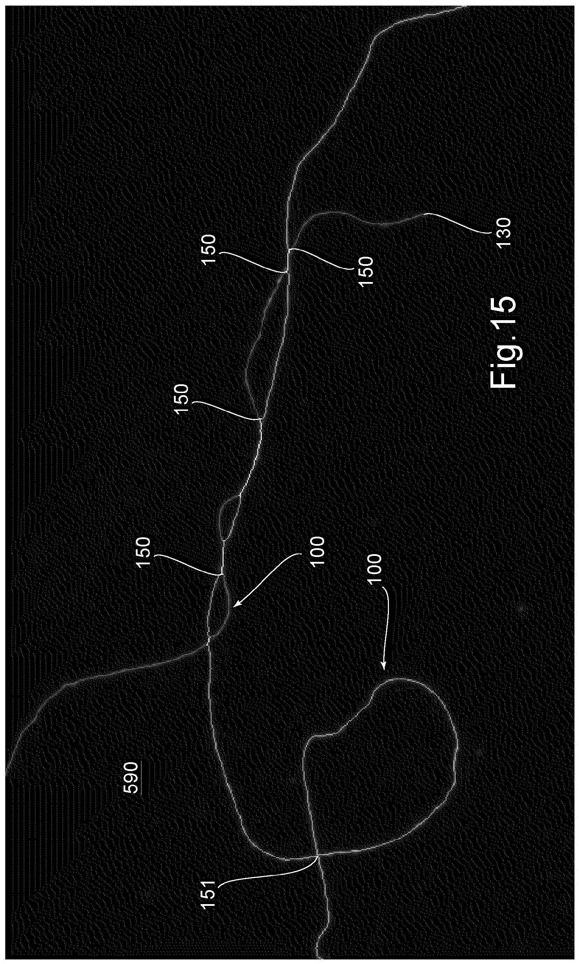

In fact, since the number of occurrences of said set of characteristics and/or said at least one structural feature may have been entered in said at least one list **120, 121,** rather in said first list **121,** a mean calculation may calculate **595** the average of said at least one fiber **911,** of said group of segments **100,** of said at least one segment **101,** of said at least one section, of said at least one structural feature, of said endpoint **130,** of said vertex **140** and/or of said junction **150,** of said at least one segment **101,** of said length of said at least one fiber **911,** of said length of said group of segments **100,** of said length of said at least one segment **101,** of said length of said at least one section and/or of said length of at least one segment **101** as shown in fig. 15 for example. Further, in fig. 15 for instance, said method **500** may distinguish two different said at least one fibers **911** and seven connections **150, 151** including six said junctions **150** and one said intersection **151** for example. In the specific case of fig. 15, said method may differentiate two different said at least one fibers **911** with six said junctions **150.** In the particular case of fig. 15, said method may recognize different connections **150, 151,** but thanks to the creation **570** of said first lists **121** having said set of characteristics **125** and said second list **122** referring to said first lists **121,** it may possible to associate said at least one lists **120** of at least one fiber **911** such as to retrace **590** individually said fiber **911** in accordance with said set of characteristics **125.** Indeed, whereas, an intersection **151** may result from a loop of said at least one fiber, as it may be particularly shown in fig. 15, only three sections may be represented **540** and said method **500** may recognize that said three sections **102** may belong to one fiber thanks to said sets of characteristics **125.**

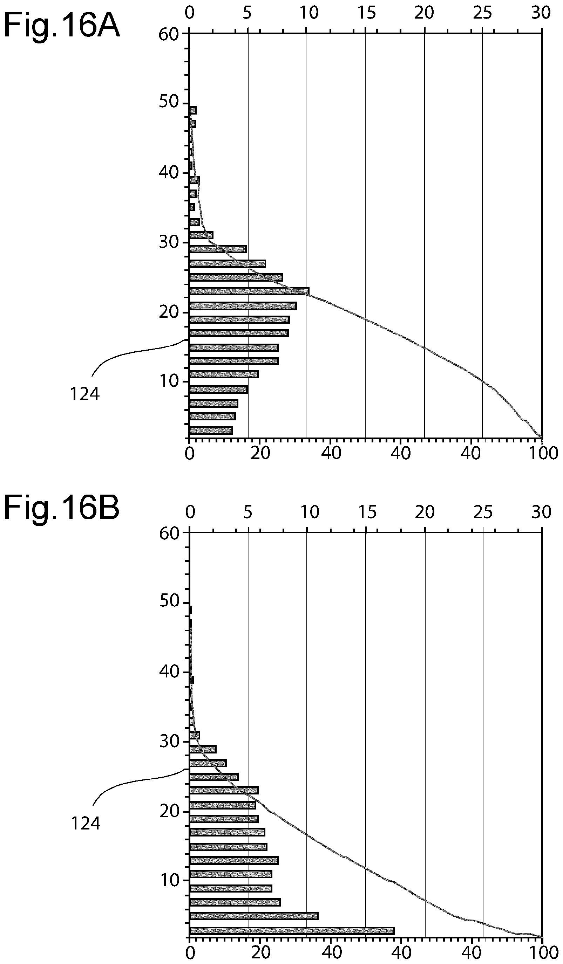

The example of figs. 16A & 16B may have been realized with fifty samples, that mean fifty acquired image **511** and 1367 at least one fiber **911.** In fig. 16A, said at least one fiber **911** may be classified by weight and the average length may be about 19.7 mm with a coefficient of variation around 41.6%. The sample may also comprise circa 20.4% fiber shorter than 12.7 mm and an upper quartile around 24.6 mm. Thanks to another aspect of the invention, the fibers may be classified by number and may offer another perspective. With the same data, the sample may comprise said at least one fiber **911** with an average length about 13.1 mm with a coefficient of variation around 65.9%. The sample may also comprise circa 53.0% fiber shorter than 12.7 mm and 5% of said at least one fiber **911** may have a length higher than 27.8 mm.

## Claims

1. Method (500) of classification of cotton fiber in a fiber layer (900); said fiber layer (900) is spread on a surface such as to acquire (510) an image (511) of said fiber layer (900) comprising a plurality of fibers (910) including at least one fiber (911); said method (500) comprising a/an:
- Application (530) of at least one image-filter (531) to said acquired image (511);
- Representation (540) of said at least one fiber (911) comprising a set of characteristics (125) by a group of segments (100) comprising at least one segment (101), at least one section and/or at least one structural feature;
- Creation (570) of at least one list (120) per said set of characteristics (125), per said at least one segment (101), per said at least one section, and/or per said at least one structural feature;
- Incrementation (520) of said at least one list (120) for each occurrence of said set of characteristics (125), said at least one segment (101), said at least one section, and/or said at least one structural feature.

2. Method (500) according to claim 1, which comprises a length calculation (595); said length calculation (595) calculates (595) the length (124) of said at least one segment (101) and/or of said at least one section of said at least one list (120).

3. Method (500) according to claim 1 or 2, which comprises a concatenation (596) of said at least one list (120) in accordance with said set of characteristics (125) such as to calculate (595) the length (124) of said at least one fiber (911).

4. Method (500) according to any of the preceding claims 1 to 3, which comprises a selection (597) of the two longest lengths of said at least one lists (120).

5. Method (500) according to any of the preceding claims 1 to 4, which comprises a mean calculation of said at least one fiber (911), of said group of segments (100), of said at least one segment (101), of said at least one section, of said at least one structural feature, of said endpoint (130), of said vertex (140) and/or of said junction (150), of said at least one segment (101), of said length of said at least one fiber (911), of said length of said group of segments (100), of said length of said at least one segment (101), of said length of said at least one section and/or of said length of at least one segment (101).

6. Method (500) according to any of the preceding claims 1 to 5, wherein said at least one segment (101) comprises extremities (110); said extremity (110) comprises an endpoint (130), a vertex (140) or a junction (150); said at least one structural feature comprises:
- said endpoint (130): said endpoint (130) terminates said at least one segment (101);
- said vertex (140); said extremities of two segments (101) meet at said vertex (140); and/or,
- said junction (150): said extremities of at least three segments (101) meet each other at said junction (150).

7. Method (500) according to any of the preceding claims 1 to 6, wherein set of characteristics (125) comprises a maturity index, a fineness, said length, a weight, a color, a waviness, a width, a spinning consistency, a uniformity index, a short fiber index, an elongation, a moisture, a reflectance, a yellowness and/or a strength.
